# EUROPEAN PATENT APPLICATION

(11) **EP 4 406 540 A1**
(43) Date of publication of application: **31.07.2024**
(21) Application number: 22873139.4
(22) Date of filing: 15.09.2022
(51) Int. Cl.: A61K 31/575, A61K 45/06, A61P 31/14

(54) **COMPOSITION FOR TREATING CORONAVIRUS DISEASE 2019 (COVID-19) CONTAINING TAURODEOXYCHOLIC ACID OR PHARMACEUTICALLY ACCEPTABLE SALT THEREOF AND ANTIVIRAL AGENT AS ACTIVE INGREDIENTS**

(30) Priority: 24.09.2021 KR 20210126676
(71) Applicant: Shaperon Inc., Seoul 06373 (KR)
(72) Inventor: SEONG, Seung Yong, Seoul 05507 (KR); LEE, Eun Ho, Yongin-si, Gyeonggi-do 16918 (KR)
(74) Representative: Vossius & Partner Patentanwälte Rechtsanwälte mbB
(86) International application number: PCT/KR2022/013768
(87) International publication number: WO 2023/048434

(57) **Abstract**

The present invention relates to a composition for treating coronavirus infection-19 (COVID-19), comprising taurodeoxycholic acid (TDCA) or a pharmaceutically acceptable salt thereof and an antiviral agent as active ingredients, it was confirmed that clinical symptoms were cured in patients with COVID-19 pneumonia who were administered a combination of a pharmaceutically acceptable salt of taurodeoxycholic acid as an inflammasome inhibitor and an antiviral agent, and thus this combination of taurodeoxycholic acid (TDCA) or a pharmaceutically acceptable salt thereof as an inflammasome inhibitor and an antiviral agent, holds promise as active ingredients in compositions for the prevention or treatment of lower respiratory tract infectious diseases, including COVID-19.

## Description

### Technical Field

The present invention relates to a composition for treating coronavirus infection-19 (COVID-19) containing taurodeoxycholic acid or a pharmaceutically acceptable salt thereof and an antiviral agent as active ingredients, in more detail, the present invention relates to a pharmaceutical composition for the prevention or treatment of lower respiratory infectious diseases, including coronavirus disease-19 (COVID-19), containing an inflammasome inhibitor including taurodeoxycholic acid or a pharmaceutically acceptable salt thereof and an antiviral agent, as active ingredients.

### Background Art

Coronavirus disease 2019 (COVID-19) is a pandemic that seriously threatens human health worldwide, with more than 200 million confirmed cases and more than 4 million deaths since its existence was first confirmed in 2019. COVID-19 is a severe acute respiratory syndrome disease spread by the novel coronavirus (SARS-CoV-2). SARS-CoV-2 belongs to the family Coronaviridae and is known to be a virus similar to SARS-CoV (Severe Acute Respiratory Syndrome Coronavirus) and MERS-CoV (Middle East Respiratory Syndrome Coronavirus). The host that is the clear source of infection and the infection route have not yet been confirmed, but it has been reported that infection is likely through contact with bats and that transmission through close contact between people is possible.

SARS-CoV-2 is a virus that can infect humans and various animals and is an RNA virus with a gene size of 27 to 32 kb. SARS-CoV-2 has an incubation period of about two weeks and mainly causes respiratory symptoms such as cough accompanied by fever, difficulty in breathing, shortness of breath, and phlegm, and in addition to these, may also cause headache, chills, runny nose, and muscle pain as well as digestive symptoms such as loss of appetite, nausea, vomiting, abdominal pain, and diarrhea.

Additionally, SARS-CoV-2 causes lower respiratory tract infections. Lower respiratory tract infections are diseases that develop when pathogens invade the respiratory tract located below the vocal cords, such as bronchitis, pneumonia, and pulmonary suppuration, and pneumonia is a representative lower respiratory tract infection. When pneumonia develops, the lungs become inflamed and the normal function of the lungs is impaired, resulting in pulmonary symptoms and systemic symptoms throughout the body. Pulmonary symptoms include coughing due to irritation of the respiratory system, phlegm due to discharge of inflammatory substances, and difficulty in breathing due to impaired breathing function, and digestive symptoms such as nausea, vomiting, and diarrhea may also occur. Additionally, systemic diseases throughout the body, such as headaches, fatigue, muscle pain, and joint pain, may develop.

SARS-CoV-2 may characteristically cause cytokine release syndrome or cytokine storm. Cytokine storm is a systemic inflammatory syndrome that may be caused by cancer, autoimmune disease, infectious disease, and the like and is characterized by excessive secretion of cytokines and hyperactivation of immune cells. This cytokine storm triggers a systemic immune response, resulting in high fever, lymph gland dysfunction, liver and spleen enlargement, cytopenia, hyperferritinosis, and central nervous system abnormalities, and may progress to multiple organ failure when left untreated. Cytokine storm also causes secondary dysfunction of various organs due to a hypersensitivity reaction of the systemic immune system, leading to patient death (Fajgenbaum et, al. N Engl J Med. 2020 Dec 3;383(23):2255- 2273). Currently, organ failure due to cytokine storm caused by COVID-19 has been identified as the leading cause of death, and pathologically, the main cause of cytokine storm is excessive activation of the NLRP3 inflammasome. Currently, corticosteroid-based steroids are generally concurrently administered to the patients to prevent cytokine storm, but there are limitations due to excessive inhibition of the systemic immune system and metabolic side effects.

The hyperactivation of the NLRP3 inflammasome complex due to the activation of P2X7 receptors after SARS-CoV-2 infection is a major cause of pneumonia in COVID-19 patients. P2X7 receptors are distributed systemically in immune cells, lung epithelial cells, and central nervous system cells, and upon infection with pathogens, including SARS-CoV-2, occurs, (1) the interaction between the viral spike protein and ACE2, the viral entry receptor, (2) due to the rise in angiotensin 2, (3) the ComC regulators are activated. Additionally, the proliferation of the virus induces cell death and released ATP activates P2X7 receptors, leading to excessive activation of the NLRP3 inflammasome, and this systemic inflammatory response can lead patients to death or cause severe sequelae such as pulmonary fibrosis.

The P2X7 receptor is an ion channel receptor composed of a complex belonging to the P2Xn purine receptor family, and stimulation of P2X7 promotes extracellular release of potassium ions and facilitates the rapid movement of calcium and sodium ions into the cell. In pathological conditions such as infection, the concentration of extracellular ATP increases significantly, leading to the activation of P2X7 receptors, which in turn triggers the efflux of potassium ions, causing intracellular ion imbalance and promoting the NLPR3 inflammasome. Consequently, pro-IL-1β/18 is activated by the NLRP3 inflammasome, exacerbating cell death and inflammation in surrounding tissues.

Despite detailed understanding of the activation mechanism of the NLRP3 inflammasome by P2X7, a P2X7 antagonist has not been developed. The polymorphism of P2X7 among individuals and its various isoforms among humans serve as significant barriers to the development of P2X7 antagonists. Additionally, GPCR19 exists as a complex bound to the cell membrane with P2X7 receptors in keratinocytes, T cells, and macrophages, among others. In inflamed cells, the expression of GPCR19 decreases significantly while the expression of P2X7 receptors increases dramatically, leading to amplified inflammatory responses.

Due to the lack of specific antiviral agents developed for COVID-19 treatment so far, symptomatic supportive therapy remains the primary approach to managing patients. Medications such as corticosteroids like dexamethasone and antiviral drugs like remdesivir are used for such supportive management. However, various side effects have been reported, and their therapeutic effects are reported to be unsatisfactory. For instance, dexamethasone, a corticosteroid, is administered orally, topically, rectally, or intravenously, but it carries significant side effects such as gastric ulcers and femoral head necrosis due to long-term use. Moreover, recent research has shown that remdesivir, an antiviral drug, has been proven beneficial in 'COVID-19 hospitalized patients requiring supplemental oxygen,' but it does not improve mortality rates as expected (N Engl J Med 2020;383:1813-26). Instead, clinical trials have been conducted using existing antiviral drugs such as lopinavir-ritonavir or favipiravir for the treatment of COVID-19 patients, but no cases proving their effectiveness have been demonstrated (Nature. 2020 May;581(7809) :465-469) .

This is due to the limitation of the therapeutic effect, as it inhibits viral replication but does not suppress the systemic inflammation already caused by the virus. To address this, a combination therapy of remdesivir and anti-inflammatory agents has been proposed in clinical settings, and some clinical efficacy has been observed (Coronavirus Disease 2019 Treatment Guidelines, US NIH, https://www.covid19treatmentguidelines.nih.gov/), however, there have been no cases demonstrating revolutionary effects thus far.

As a result of efforts to develop a combination therapy that demonstrates excellent therapeutic effects, safety, and minimal side effects for lower respiratory tract infectious diseases caused by coronaviruses such as SARS-CoV-2, the inventors have confirmed the treatment of clinical symptoms in patients with COVID-19 pneumonia by administering a combination of taurodeoxycholic acid, or its pharmaceutically acceptable salt, and antiviral agents, aiming to simultaneously inhibit the activation of the NLRP3 inflammasome and suppress systemic inflammatory responses and viral replication induced by COVID-19 and so this application discloses the utility of taurodeoxycholic acid or its pharmaceutically acceptable salt, in combination with antiviral agents, as effective components of combination preparations for the prevention or treatment of lower respiratory tract infectious diseases, including COVID-19.

### Summary of Invention

### Technical Problem

Antiviral agents used to the treatment of COVID-19 have shown limitations as monotherapy, so combination therapy is needed to complement them.

Accordingly, the purpose of the present invention is to provide a complex, mixture or combination preparations for use in preventing or treating lower respiratory tract infectious diseases such as COVID-19.

In order to achieve the object of the present invention, the present invention provides a pharmaceutical composition for the prevention or treatment of lower respiratory tract infectious diseases comprising a first component containing an inflammasome inhibitor; and a second component containing at least one antiviral agent as an active ingredient in a complex, mixture or combination preparation for use in the prevention or treatment of lower respiratory tract infectious diseases.

Furthermore, the present invention provides a method for the prevention or treatment of lower respiratory tract infectious diseases, comprising the step of administering a complex, mixture, or combination preparation comprising a first component comprising an inflammasome inhibitor; and a second component containing at least one antiviral agent in pharmacologically effective amounts to a subject.

Additionally, the present invention provides a use of a complex, mixture, or combination preparation comprising a first component containing an inflammasome inhibitor; and a second component containing at least one antiviral agent as an active ingredient for use as a pharmaceutical composition for preventing or treating lower respiratory tract infectious disease.

Additionally, the present invention provides a use of a complex, mixture, or combination preparation comprising a first component containing an inflammasome inhibitor; and a second component containing at least one antiviral agent as an active ingredient for the manufacture of pharmaceutical composition for the prevention or treatment of lower respiratory tract infectious diseases.

Moreover, the present invention provides a kit comprising a complex, mixture, or combination preparation for the prevention or treatment of lower respiratory tract infectious diseases, comprising a preparation containing an inflammasome inhibitor; and a preparation containing one or more antiviral agents.

### Advantageous Effects of Invention

The inventors confirmed the therapeutic effect of administering a combination of taurodeoxycholic acid, or its pharmaceutically acceptable salt, and antiviral agents to patients with COVID-19 pneumonia by inhibiting the activation of the NLRP3 inflammasome, thereby simultaneously suppressing systemic inflammatory responses and virus proliferation induced by COVID-19, and so the inflammasome inhibitor including taurodeoxycholic acid or its pharmaceutically acceptable salt, and antiviral agents, can be utilized as effective components of a complex, mixture, or combination preparation for lower respiratory tract infectious diseases, including COVID-19.

### Brief Description of Drawings

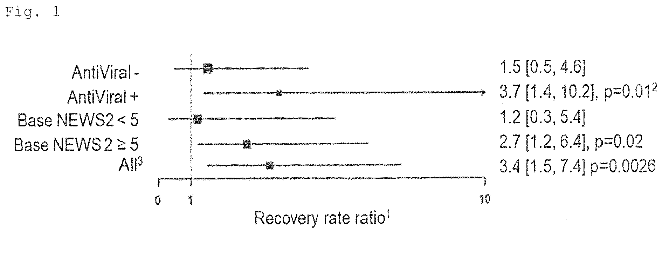

Figure 1 shows the case where antiviral agents (lopinavir/ritonavir or favipiravir) were co-administered as a baseline factor among compliant trial patients (PP) in a clinical trial targeting COVID-19 pneumonia patients and the results of Cox-regression analysis of the recovery rate ratio (RRR) adjusted statistically for the severity of moderate to severe cases with NEWS2 scores of 5 or higher. In this figure, ¹ indicates the recovery rate ratio (RRR), ² represents the p-value, which is determined by the Cox proportional hazards model, a RRR > 1 indicates a favorable recovery rate in the NuSepin treatment group, and ³ depicts the analysis results adjusting for both antiviral drug treatment and severity in all PP patients.

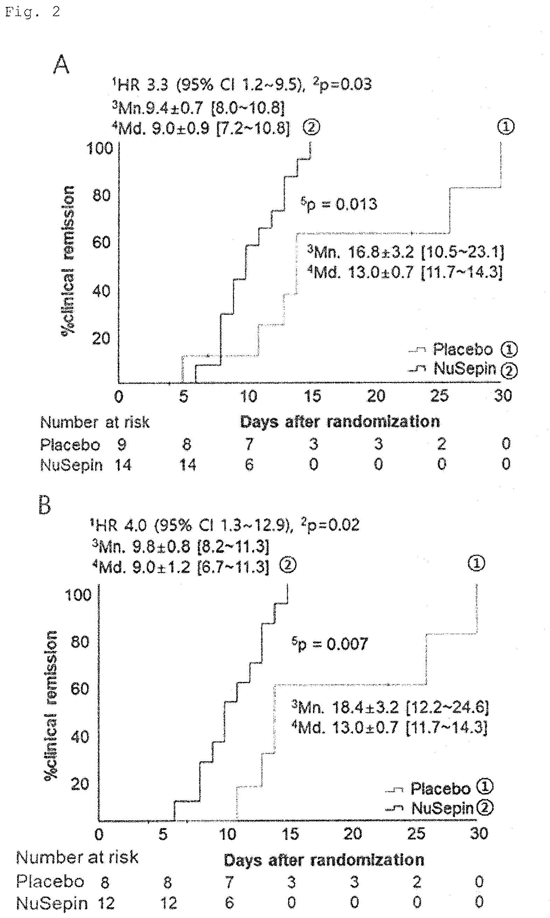

Figure 2 shows a diagram comparing clinical remission time with the time taken to reach NEW2=0 using Kaplan-Meier survival analysis, in the subset of patients receiving NuSepin and antiviral agents such as lopinavir/ritonavir or favipiravir among the entire clinical trial population (Figure 2A) or among the subset of COVID-19 clinical patients with moderate to severe illness (NEWS2 score ≥ 5) (Figure 2B). Here, - represents the Hazard Ratio, ² represents the p-value, which is determined by the Cox proportional hazards model, a Hazard Ratio > 1 indicates a favorable recovery rate in the NuSepin treatment group, ³ represents the mean ± standard error [95% C.I.], ¹ represents the median ± standard error [95% C.I.], and ⁵ represents the p-value, which is derived from the Log-Rank test (Mantel-Cox) of the Kaplan-Meier survival analysis.

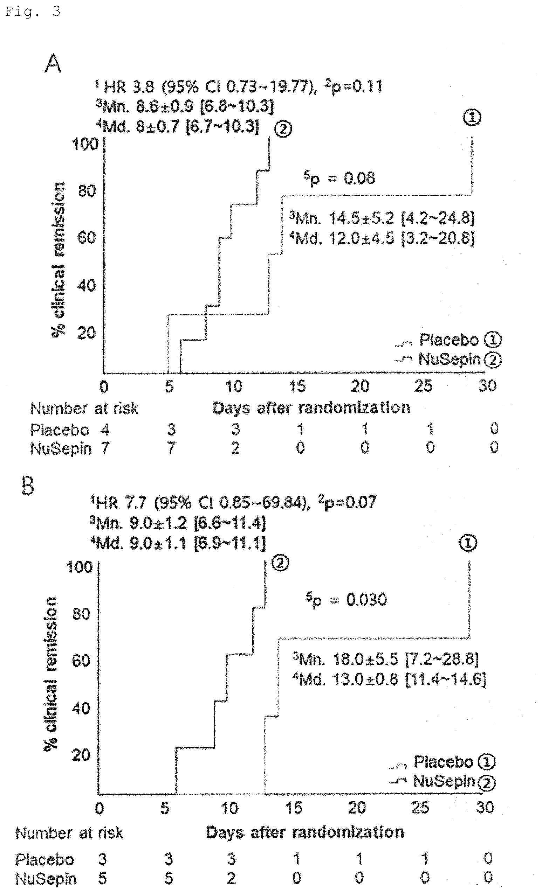

Figure 3 shows a diagram comparing clinical remission time with the time taken to reach NEW2=0 using Kaplan-Meier survival analysis, in the subset of patients receiving NuSepin and antiviral agents such as lopinavir/ritonavir among the entire clinical trial population (Figure 3A) or among the subset of COVID-19 clinical patients with moderate to severe illness (NEWS2 score ≥ 5) (Figure 3B). Here, ¹ represents the Hazard Ratio, ² represents the p-value, which is determined by the Cox proportional hazards model, a Hazard Ratio > 1 indicates a favorable recovery rate in the NuSepin treatment group, ³ represents the mean ± standard error [95° C.I.], ⁴ represents the median ± standard error [95% C.I.], and ⁵ represents the p-value, which is derived from the Log-Rank test (Mantel-Cox) of the Kaplan-Meier survival analysis.

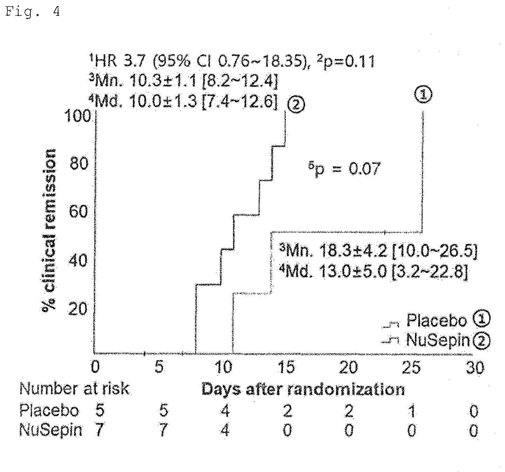

Figure 4 shows a diagram comparing clinical remission time with the time taken to reach NEW2=0 using Kaplan-Meier survival analysis, in the subset of patients receiving NuSepin and favipiravir as an antiviral agent among the entire clinical patient group (ITT) in a clinical trial for patients with COVID-19 pneumonia. Here, ¹ represents the Hazard Ratio, ² represents the p-value, which is determined by the Cox proportional hazards model, a Hazard Ratio > 1 indicates a favorable recovery rate in the NuSepin treatment group, ³ represents the mean ± standard error [95% C.I.], ⁴ represents the median ± standard error [95% C.I.], and ⁵ represents the p-value, which is derived from the Log-Rank test (Mantel-Cox) of the Kaplan-Meier survival analysis.

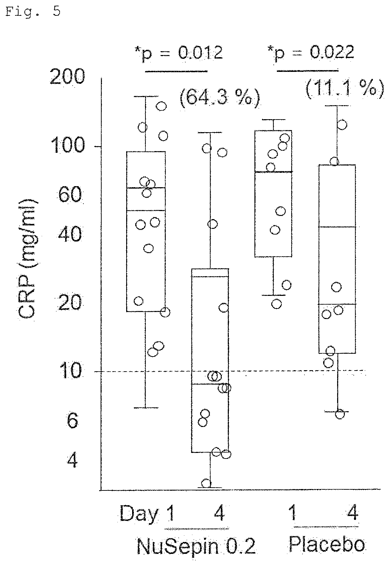

Figure 5 shows a diagram comparing changes in CRP between the patient group administered in combination with NuSepin 0.2 mg/kg and antiviral agents and the placebo group in a clinical trial for patients with COVID-19 pneumonia. Here, the % in parentheses represents the % of patients with normal blood levels (CRP < 10 mg/ml) on day 4, and the p-value in * is determined by the Wilcoxon Rank sum test, and p-value < 0.05 considered statistically significant.

### Description of Embodiments

Hereinafter, the present invention will be described in more detail.

In the present invention, the term "prevention" refers to all actions to suppress or delay the occurrence, spread, and recurrence of lower respiratory tract infectious diseases by administering the composition of the present invention, and the term "treatment" refers to all actions to improve or beneficially change the symptoms of the disease by administration of the composition of the present invention.

In the present invention, the term "administration" means providing a predetermined substance to a patient by any appropriate method, and the route of administration of the composition of the present invention is oral or parenteral through all general routes as long as it can reach the target tissue. Additionally, the composition can be administered by any device capable of transporting the active agent to target cells.

The present invention provides a pharmaceutical composition for the prevention or treatment of lower respiratory tract infectious diseases comprising: a first ingredient containing an inflammasome inhibitor; and a second component containing at least one antiviral agent as an active ingredient in a complex, mixture, or combination preparation.

Furthermore, the present invention provides a method for the prevention or treatment of lower respiratory tract infectious diseases, comprising the step of administering a complex, mixture, or combination preparation comprising a first component comprising an inflammasome inhibitor; and a second component containing at least one antiviral agent in pharmacologically effective amounts to a subject.

Additionally, the present invention provides a use of complex, mixture, or combination preparation comprising a first component comprising an inflammasome inhibitor; and a second component containing at least one antiviral agent for the prevention or treatment of lower respiratory tract infectious diseases.

Additionally, the present invention provides a use of complex, mixture, or combination preparation comprising a first component comprising an inflammasome inhibitor; and a second component containing at least one antiviral agent for the manufacture of pharmaceutical composition for the prevention or treatment of lower respiratory tract infectious diseases.

Moreover, the present invention provides a kit comprising a complex, mixture, or combination preparation for the prevention or treatment of lower respiratory tract infectious diseases, comprising a preparation containing an inflammasome inhibitor; and a preparation containing one or more antiviral agents.

In the present invention, the term "inflammasome inhibitor" refers to an NLRP3 inflammasome inhibitor, which can inhibit the formation or activation of the NLRP3 inflammasome.

Specifically, the inflammasome inhibitor may be taurodeoxycholic acid or its pharmaceutically acceptable salt.

The taurodeoxycholic acid is a type of bile acid and is in the form of taurine-conjugated deoxycholic acid, and has a chemical structure described in the following [Formula 1], and more specifically, a chemical structure represented by the following [Formula 2]:

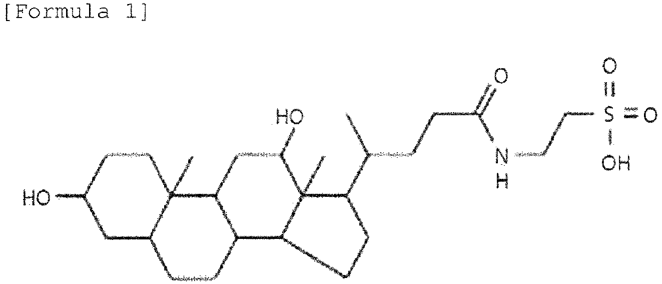

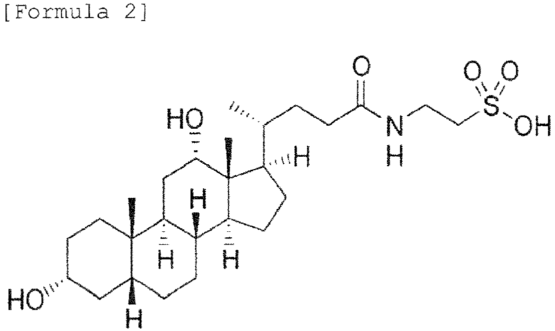

Additionally, taurodeoxycholic acid can be sourced from animal carcasses, such as cattle, pigs, sheep, dogs, goats, or rabbits, obtained commercially, or synthesized, all of which are acceptable for use.

In the present invention, the inflammasome inhibitor may be administered at a dosage ranging from 0.01 to 1 mg/kg/day (mg/kg of body weight/day), 0.05 to 0.5 mg/kg/day (mg/kg of body weight/day), 0.07 to 0.3 mg/kg/day (mg/kg of body weight/day), or 0.1 to 0.2 mg/kg/day (mg/kg of body weight/day). Alternatively, it may be administered at dosages of 0.01, 0.05, 0.07, 0.1, 0.2, 0.3, 0.5, or 1 mg/kg/day (mg/kg of body weight/day).

The antiviral agent according to the present invention may be an RNA-dependent RNA polymerase inhibitor (RdRp inhibitors) category compound, a derivative thereof, or a pharmaceutically acceptable salt thereof, and the RdRp inhibitor category compound is selected from the group consisting of remdesivir, favipiravir, chlorhexidine, novobiocin, ceftibuten, ribavirin, atovaquone, valganciclovir, cromolyn, bromocriptine, silybin, cefuroxime, fludarabine, galidesivir, sofosbuvir, fenoterol, nitazoxanide, itraconazole, benzylpenicilloyl G, pancuronium bromide, penciclovir, 7-deza-2'-C-methyladenosine, idarubicin, diphenoxylate, ganciclovir, tenofovir, tibolone, chenodeoxycholic acid and cortisone.

In addition, the antiviral agent according to the present invention may be a protease inhibitor (PIs) category compound or a derivative thereof, or a pharmaceutically acceptable salt thereof, and the PI category compound or a derivative thereof may be selected from the group consisting of single agents such as saquinavir, ritonavir, indinavir, nelfinavir, amprenavir, lopinavir, atazanavir, fosamprenavir, tipranavir, darunavir, and combination therapies including atazanavir+cobicistat, darunavir+cobicistat, ritonavir+lopinavir.

In addition, the antiviral agent according to the present invention may be a non-nucleoside reverse-transcriptase inhibitors (NNRTIs) category compound or a derivative thereof, or a pharmaceutically acceptable salt thereof, and the NNRTI category compound or a derivative thereof may be selected from the group consisting of efavirenz, etravirine, nevirapine, doravirine, rilpivirine, and delavirdine.

In addition, the antiviral agent according to the present invention may be a nucleoside reverse-transcriptase inhibitors (NRTIs) category compound or a derivative thereof, or a pharmaceutically acceptable salt thereof, and the NRTI category compound or a derivative thereof may be selected from the group of zidovudine, didanosine, zalcitabine, stavudine, lamivudine, abacavir, tenofovir disoproxil fumarate, emtricitabine, tenofovir alafenamide fumarate.

In addition, the antiviral agent according to the present invention may be an Integrase Strand Transfer Inhibitor (INSTI) category compound or a derivative thereof, or a pharmaceutically acceptable salt thereof, and the INSTI category compound or a derivative thereof may be a dolutegravir or raltegravir.

Additionally, the antiviral agent according to the present invention may be a fusion inhibitor category compound, a derivative thereof, or a pharmaceutically acceptable salt thereof, and the fusion inhibitor may be enfuvirtide.

In addition, the antiviral agent according to the present invention may be a CCR 5 (Chemokine (C-C motif) ligand 5) inhibitor category compound or a derivative thereof, or a pharmaceutically acceptable salt thereof, and the CCR 5 inhibitory compound or a derivative thereof may be selected from the group consisting of single agents such as maraviroc and combination therapies selected from the group consisting of cobicistat/elvitegravir/emtricitabine/Tenofovir alafenamide, cobicistat/Elvitegravir/emtricitabine/Tenofovir disoproxil, and abacavir/dolutegravir/lamivudine.

In addition, the antiviral agent according to the present invention may be a flu therapeutic agentused for the treatment of influenza A and influenza B virus infections, and the flu treatment may be selected from the group consisting of an uncoating inhibitor category compound or a derivative thereof, or a pharmaceutically acceptable salt thereof, such as amantadine, rimantadine and neuraminidase inhibitors (NAIs) category compound or derivatives thereof, or pharmaceutically acceptable salts thereof such as oseltamivir and zanamivir, peramivir, baloxavir and laninamivir.

In addition, the antiviral agent according to the present invention may be a herpes therapeutic agent served as the treatment of herpes simplex virus (HSV) and varicella zoster virus (VZV) infections, and the herpes therapeutic agent may be selected from the group consisting of aciclovir, valaciclovir, idoxuridine, vidarabine, penciclovir, famciclovir, trifluridine, cidofovir and foscarnet.

Furthermore, the antiviral agent according to the present invention may function as a therapeutic agent for hepatitis B virus (HBV), inhibiting its proliferation and thereby relieving inflammation, preventing fibrosis, and reducing the risk of cirrhosis and hepatocellular carcinoma, and the HBV therapeutic agent may be selected from the group consisting of lamivudine, clevudine, telbivudine, entecavir, adefovir, tenofovir disoproxil, tenofovir alafenamide, and besifovir. In cases of resistance, switching to alternative medications or transitioning to combination therapy with two drugs may be considered

In addition, the antiviral agent according to the present invention may be a hepatitis C therapeutic agent that delays the progression of the disease by inhibiting the proliferation of the hepatitis C virus, and the hepatitis C therapeutic agent may be selected from the group consisting of a cytokine such as peginterferon-α-2a and peginterferon-α-2b, a compound in the class of antivirals (DAA; Direct-acting antivirals) that acts on viral proteins or a derivative thereof, or a pharmaceutically acceptable salt thereof, such as boceprevir, dasabuvir, daclatasvir as a single agent, and elbasvir/grazoprevir, glecaprevir/pibrentasvir, and ombitasvir/paritaprevir/ritonavir as combination therapy.

In the present invention, the antiviral agent may be administered in a dosage ranging from 0.01 to 5000 mg/kg/day (mg/kg of body weight/day), 0.05 to 4000 mg/kg/day (mg/kg of body weight/day), 0.07 to 3000 mg/kg/day (mg/kg of body weight/day) or 0.1 to 2000 mg/kg/day (mg/kg of body weight/day).

In the present invention, the content of the inflammasome inhibitor and one or more antiviral agents of the present invention may be appropriately selected depending on the dosage form, and the like.

When the inflammasome inhibitor and one or more antiviral agents are formulated into a single preparation, the content of the inflammaxome inhibitor is generally about 0.01 to about 99.99 wt% of the total preparation, specifically about 0.01 to about 90 wt%, preferably about 0.1 to about 90 wt%, more preferably about 0.1 to about 80 wt%, even more preferably about 0.1 to about 70 wt% based on the entire preparation, and similarly, the content of one or more antiviral agents is generally about 0.01 to about 99.99 wt% of the total preparation, specifically about 0.01 to about 90 wt%, preferably about 0.1 to about 80 wt%, more preferably about 0.1 to about 70 wt%, and even more preferably about 0.1 to about 60 wt% based on the entire preparation.

When formulated into a single preparation, the mixing ratio of the inflammasome inhibitor and one or more antiviral agents of the present invention may range from about 0.00001 to 100 parts by weight of the first component (inflammasome inhibitor) to 1 part by weight of the second component (one or more antiviral agents), or specifically, from about 0.00001 to 90 parts by weight of the first component to 1 part by weight of the second component, or from about 0.00001 to 80 parts by weight of the first component to 1 part by weight of the second component, from about 0.00001 to 70 parts by weight of the first component to 1 part by weight of the second component, or from about 0.00001 to 60 parts by weight of the first component to 1 part by weight of the second component, from about 0.00001 to 50 parts by weight of the first component to 1 part by weight of the second component, or from about 0.00001 to 40 parts by weight of the first component to 1 part by weight of the second component, from about 0.00001 to 30 parts by weight of the first component to 1 part by weight of the second component, or from about 0.00001 to 20 parts by weight of the first component to 1 part by weight of the second component, from about 0.00001 to 10 parts by weight of the first component to 1 part by weight of the second component, or from about 0.00001 to 1 part by weight of the first component to 1 part by weight of the second component.

On the other hand, when the inflammasome inhibitor and one or more antiviral agents are separately formulated and combined, the content of additives such as excipients may vary, but generally ranges from about 1 to 99.00 wt% for each formulation, or specifically from about 1 to about 90 wt%, or preferably from about 10 to about 90 wt%, more preferably from about 10 to about 80 wt%, or even more preferably from about 10 to about 70 wt%.

In the present invention, the lower respiratory tract infectious disease may be a disease having one or more symptoms selected from the group consisting of fever, cough, phlegm, body aches, sore throat, diarrhea, conjunctivitis, headache, skin rash, difficulty in breathing, acute bronchitis, pneumonia, pulmonary suppuration, renal failure, renal dysfunction, septic shock, sepsis, and cytokine release syndrome caused by coronavirus infection, but is not limited thereto.

The coronavirus may be one or more selected from the group consisting of Middle East respiratory syndrome coronavirus (MERS-CoV), severe acute respiratory syndrome coronavirus (SARS-CoV), and novel severe acute respiratory syndrome coronavirus 2 (SARS-CoV-2), specifically may be novel severe acute respiratory syndrome coronavirus 2 (SARS-CoV-2), but is not limited thereto.

The present invention includes not only taurodeoxycholic acid but also its pharmaceutically acceptable salts and possible solvates, hydrates, racemates, or stereoisomers that can be prepared therefrom. Additionally, an antiviral agent according to the present invention includes its pharmaceutically acceptable salts and possible solvates, hydrates, racemates, or stereoisomers that can be prepared therefrom.

Compound according to the present invention can be used in the form of a pharmaceutically acceptable salt, and an acid addition salt formed from a pharmaceutically acceptable free acid is useful as the salt. Acid addition salts are obtained from inorganic acids such as hydrochloric acid, nitric acid, phosphoric acid, sulfuric acid, hydrobromic acid, hydroiodic acid, nitrous acid or phosphorous acid, and non-toxic organic acids such as aliphatic mono- and dicarboxylates, phenyl-substituted alkanoates, hydroxyalkanoates and alkanedioates, aromatic acids, and aliphatic and aromatic sulfonic acids. These pharmaceutically non-toxic salts include a sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methyl benzoate, dinitro benzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, hydroxybutyrate, glycolate, malate, tartrate, methanesulfonate, propanesulfonate, naphthalene-1-sulfonate, naphthalene-2-sulfonate, or mandelate.

The acid addition salt according to the present invention can be prepared by conventional methods, for example, by dissolving taurodeoxycholic acid in an excess of aqueous acid solution and precipitating the salt using a water-miscible organic solvent, such as methanol, ethanol, acetone, or acetonitrile. The acid addition salt can also be prepared by evaporating the solvent or excess acid from this mixture for drying, or suction filtering the precipitated salt.

A pharmaceutically acceptable metal salt can be prepared using a base. An alkali metal or alkaline earth metal salt is obtained, for example, by dissolving the compound in an excess of alkali metal hydroxide or alkaline earth metal hydroxide solution, filtering the undissolved compound salt, and evaporating the filtrate for drying. At this time, it is pharmaceutically appropriate to prepare a sodium, potassium, or calcium salt as the metal salt, and more specifically, it is appropriate to prepare a sodium salt. The corresponding silver salt is obtained by reacting the alkali metal or alkaline earth metal salt with a suitable silver salt (for example, silver nitrate).

When the composition is formed into a preparation, the preparation is prepared using diluents or excipients such as commonly used fillers, extenders, binders, wetting agents, disintegrants, and surfactants.

In the present invention, the formulation may be formulated into various dosage forms selected from the group consisting of tablets, capsules, injections, troches, powders, granules, solutions, suspensions, oral solutions, emulsions, syrups, suppositories, vaginal tablets, and pills, but is not limited thereto, and it can be formulated into an appropriate formulation as needed.

For example, preparations for oral administration include tablets, pills, powders, granules, capsules, troches, and the like, and these solid preparations are prepared by mixing compound or a pharmaceutically acceptable salt thereof of the present invention with at least one or more excipients, such as starch, calcium carbonate, sucrose or lactose, or gelatin. In addition to simple excipients, lubricants such as magnesium stearate, and talc are also used. Liquid preparations for oral administration include suspensions, oral solutions, emulsions, or syrups, and may contain various excipients such as wetting agents, sweeteners, fragrances, and preservatives in addition to commonly used simple diluents such as water and liquid paraffin.

Preparations for parenteral administration include sterilized aqueous solutions, non-aqueous solutions, suspensions, emulsions, freeze-dried preparations, suppositories, and the like.

As non-aqueous solvents and suspending agents, propylene glycol, polyethylene glycol, vegetable oil such as olive oil, and injectable esters such as ethyl oleate may be used. As base materials for suppositories, witepsol, macrogol, tween 61, cacao oil, laurin oil, glycerol, gelatin, and the like may be used.

The composition according to the present invention is administered in a pharmaceutically effective amount. In the present invention, the "pharmaceutically effective amount" means an amount sufficient to treat a disease with a reasonable benefit/risk ratio applicable to medical treatment, and the effective dose level may be determined based on factors including the type and severity of the patient's disease, activity of the drug, sensitivity to the drug, time of administration, route of administration and excretion rate, treatment period, and drugs used concurrently, and other factors well known in the medical field. The composition of the present invention may be administered as an individual therapeutic agent or concurrently with other therapeutic agents, may be administered sequentially or simultaneously with conventional therapeutic agents, and may be administered singly or multiple times. It is important to administer the composition of the present invention in an amount so that the maximum effect can be acquired with the minimum amount without side effects, taking into account all of the above factors, and this may be easily determined by those skilled in the art.

Specifically, the effective amount of the compound according to the present invention may vary depending on the patient's age, gender, and weight, and may be administered daily or every other day, or divided into 1 to 3 times per day. However, since it may increase or decrease depending on the route of administration, severity, gender, weight, age, etc., the above dosage does not limit the scope of the present invention in any way.

In the present invention, a first component containing an inflammasome inhibitor; and a second component comprising one or more antiviral agents are administered to a subject in need of treatment for a lower respiratory tract infectious disease in a mixed, complexed, or combined form.

In a specific embodiment of the present invention, the present inventors confirmed the treatment of lower respiratory tract infectious diseases using a complex, mixture, or combination administration of an inflammasome inhibitor and antiviral agents, and they observed significant clinical symptom relief when administering combination therapy of a taurodeoxycholic acid sodium salt as the inflammasome inhibitor with a protease inhibitor compounds, such as lopinavir+ritonavir, or a RdRp inhibitor compound, such as favipiravir, to COVID-19 pneumonia patients.

Therefore, the present inventors confirmed that clinical symptoms were treated in patients with COVID-19 pneumonia who were administered a combination of a pharmaceutically acceptable salt of taurodeoxycholic acid as an inflammatory complex inhibitor and an antiviral agent, and the inflammasome inhibitor and antiviral agents can be used as active ingredients in compositions for preventing or treating lower respiratory tract infectious diseases, including COVID-19, or complex, mixed, or combination drug kits for preventing or treating lower respiratory tract infectious diseases.

Hereinafter, the present invention will be described in detail through examples and experimental examples.

However, the following examples and experimental examples only illustrate the present invention, and the content of the present invention is not limited to the following examples.

### <Example 1> Examination of the efficacy of combination therapy of TDCA and antiviral agents in patients with coronavirus infection-19 (COVID-19) - Phase 2 clinical trial

### <1-1> Selection of clinical trial subjects

The subjects of the clinical trial were those who met all of the criteria in [Table 1] among those infected with coronavirus disease 2019 (COVID-19) in Romania, Europe.

**[Table 1]**

| Order | Patient selection criteria |
|---|---|
| 1 | Male or female over 18 years of age but under 80 years of age |
| 2 | A case where SARS-CoV-2 infection is first confirmed by laboratory PCR test within 144 hours prior to randomization |
| 3 | A case where radiographic images (chest x-ray, CT scan) and three of the following clinical symptoms occur (new cough, fever, fatigue, sputum (all day), tachypnea, difficulty in breathing, and pleuritic chest pain) and a case where CRP > 10mg/L or more |
| 4 | A case where the white blood cell count in the patient's blood > 4.0 × 10⁹/L and lymphocyte count > 0.7 × 10⁹/L |
| 5 | A case where indoor SpO₂ ≤ 94% or Pₐ₀₂/F_{I02} ratio < 300 mmHg during the screening stage |
| 6 | A person who determines that it is appropriate to participate in a clinical trial and gives written consent to participate in a clinical trial of his or her own free will |
| 7 | For women of childbearing age, those who agree to continue using medically acceptable effective contraception until 90 days after the last administration in the clinical trial |

However, subjects who met the items shown in [Table 2] below were excluded.

**[Table 2]**

| Order | Patient exclusion criteria |
|---|---|
| 1 | A case where ALT (Alanine Transaminase) or AST (Aspartate Transaminase) > 5xULN |
| 2 | A case where eGFR < 30 ml/min and hemodialysis or hemofiltration is required because of decreased renal function |
| 3 | Pregnant or lactating women |
| 4 | A case where there are signs of multiple organ failure |
| 5 | A case where steroid is administered 72 hours before participation in the clinical trial |
| 6 | A case where there has been participation in a clinical trial related to COVID-19 |
| 7 | A case where the doctor judges that participating in the clinical trial is not the best option for the patient or that the clinical protocol cannot be safely performed |

During the clinical trial, subjects who met the criteria in [Table 3] dropped out.

**[Table 3]**

| Order | Patient dropout criteria |
|---|---|
| 1 | A case where serious side effects occur according to the clinical investigator's judgment, including lab abnormalities |
| 2 | A case where a patient does not properly comply with the clinical trial |
| 3 | A case where serious deviations from the protocol are discovered |
| 4 | A case where treatment with prohibited drugs (steroids) is required |
| 5 | A case where a patient gets pregnant midway through the clinical trial |
| 6 | A case where the principal investigator decides to discontinue the clinical trial on a patient for any reason |

### <1-2> Progress and management of clinical trial

To evaluate the efficacy of the treatment, a randomized, double-blind, placebo-controlled trial was conducted with a total of 64 enrolled clinical trial patients. As part of the treatment, NuSepin, an injectable clinical formulation containing TDCA, was administered in combination with antiviral agents including Lopinavir-Ritonavir, Favipiravir, Remdesivir, or other antiviral drugs such as umifenovir.

### [Progress stage]

### Patient screening (Day -5 to 0)

Patients who wanted to participate in the clinical trial signed the patient information sheet and informed consent form and then underwent a screening test. A screening number was assigned to each patient, and demographic information, patient medical history, past and current medication history, current medical history, vital signs (blood pressure, heart rate, body temperature, respiratory rate, level of consciousness), physical examination, electrocardiogram, laboratory tests, COVID-19 PCR test, arterial blood gas analysis, and chest x-ray or CT scan to evaluate pulmonary infiltration were performed. For women of childbearing age, a serum pregnancy test was performed.

### Setting of patient criteria (Day 1)

After monitoring of vital signs (blood pressure, heart rate, body temperature, respiratory rate, level of consciousness), transcutaneous oxygen saturation (SpO₂), physical examination, and evaluation of clinical trial inclusion/exclusion criteria were performed. The clinical status of the patient was evaluated using a 6-level scale.

The patients were randomly assigned to one of the placebo groups that received standard treatment with a NuSepin 0.1, 0.2 mg/kg and antiviral agents (lopinavir/ritonavir 400-800 mg/100-200 mg per oral, favipiravir 1200 mg per oral, Remdesivir (100-200 mg/head), and umifenovir (200-600 mg per oral) as other antiviral drugs) as shown in [Table 4]. Subsequently, the drug was administered intravenously two times a day from day 1to day 14 or until the day of discharge. During the administration period, adverse drug reactions (AEs) and all details of the drugs administered to the patients were collected, recorded, and managed.

**[Table 4]**

| treatment arm | N, case | N, Event 1 | Censored | |
|---|---|---|---|---|
| | | | N | % |
| Patients receiving NuSepin only | | | | |
| Placebo | 10 | 9 | | 10.0% |
| NuSepin 0.1 mg/kg dosage group | 10 | 5 | 5 | 50.0% |
| NuSepin 0.2 mg/kg dosage group | 6 | 6 | 0 | 0.0% |
| Total | 26 | 20 | 6 | 23.1% |

| Patients receiving NuSepin and lopinavir/ritonavir in combination | | | | |
|---|---|---|---|---|
| Placebo | 4 | 4 | 0 | 23.1% |
| NuSepin 0.1 mg/kg dosage group | 6 | 5 | 1 | 0.0% |
| NuSepin 0.2 mg/kg dosage group | 7 | 7 | 0 | 0.0% |
| Total | 17 | 16 | 1 | 5.9% |

| Patients receiving NuSepin and Favipiravir in combination | | | | |
|---|---|---|---|---|
| Placebo | 5 | 3 | 2 | 40.0% |
| NuSepin 0.1 mg/kg dosage group | 4 | 4 | 0 | 0.0% |
| NuSepin 0.2 mg/kg dosage group | 7 | 7 | 0 | 0.0% |
| Total | 16 | 14 | 2 | 12.5% |

| Patients receiving of NuSepin and Remdesivir in combination | | | | |
|---|---|---|---|---|
| Placebo | 1 | 1 | 0 | 0% |
| NuSepin 0.1 mg/kg dosage group | 1 | 1 | 0 | 0% |
| NuSepin 0.2 mg/kg dosage group | 22 | 0 | 2 | 100% |
| Total | 4 | 2 | 2 | 50% |

| Patients receiving NuSepin and other anti-viral drug in combination | | | | |
|---|---|---|---|---|
| Placebo | 0 | 0 | 0 | 0.0% |
| NuSepin 0.1 mg/kg dosage group | 1 | 1 | 0 | 0.0% |
| NuSepin 0.2 mg/kg dosage group | 0 | 0 | 0 | 0.0% |
| Total | 1 | 1 | 0 | 0.0% |
| Total | 64 | 53 | 11 | 17.2% |
| ¹ Time to clinical improvement defined by NEWS2=0 | | | | |
| ² Two patients also received lopinavir/ritonavir or favipiravir | | | | |

### Progress of clinical evaluation (Day 2 to Day 14)

For 2 to 14 days from the test date, vital signs (blood pressure, heart rate, body temperature, respiratory rate, level of consciousness), SpO₂, and physical examination were regularly monitored, the patient's clinical status was evaluated, and laboratory tests (serum chemistry analysis, blood tests, urinalysis, liver/renal function tests, inflammatory parameters), 12-lead ECG, adverse drug events, and changes in drug administered to the patient were collected and recorded. Depending on the judgment of the researcher and clinician, the participant's early discharge was decided, and those eligible for early discharge underwent follow-up management and final completion test process to collect research data.

### Follow-up management

After 14 days of the administration experiment, the collection of vital sign monitoring (blood pressure, heart rate, body temperature, respiratory rate, level of consciousness), SpO₂ measurement, 6-scale clinical status evaluation, physical examination, 12-lead ECG, urine pregnancy test (women of childbearing age), laboratory analysis (serum chemistry, urinalysis, liver/renal function tests, inflammatory marker tests), adverse drug events, and changes in drug during administration period was completed after the administration of the study drug was completed.

### Completion of clinical trial

Finally, on day 28, a clinical follow-up visit, physical examination, SpO₂ measurement, and laboratory analysis (serum chemistry, blood tests, urinalysis, liver/renal function tests, and inflammatory marker tests) were performed to collect information on adverse drug events, changes in drug concurrently used, and survival status.

### [Clinical evaluation indicators]

### 1. Time to Clinical Improvement (TTCI)

As the primary clinical evaluation index, TTCI was assessed and recorded from days 1 to 15 and at the end date. The TTCI index is a clinical index that is divided into 6 levels and scored based on the standards in [Table 5], and the lower the score, the better the treatment. The point at which the level was lowered by two or more levels was judged to be the point of treatment and recorded.

**[Table 5]**

| Score | Criteria |
|---|---|
| 1 | Complete Clinical Remission |
| 2 | Hospitalization, not requiring supplemental oxygen |
| 3 | Hospitalization, requiring supplemental oxygen (such as LFNC or facial mask) |
| 4 | ICU/Hospitalization, requiring NIV/HFNC therapy |
| 5 | ICU, requiring ECMO and/or IMV |
| 6 | Death |

### 2. NEWS2(National Early Warning Score 2)

As a secondary clinical evaluation index, NEWS2 was evaluated and recorded from days 1 to 15 and at the end date. The NEWS2 clinical index is a clinical evaluation index that is scored based on the measurement data such as whether an oxygen respirator is needed, body temperature, blood pressure, heart rate, body temperature, respiratory rate, level of consciousness, and oxygen saturation according to the criteria in [Table 6] and summed up to evaluate the degree of the patient's disease and to evaluate/determine the treatment stage. Patients with a NEWS2 index of 7 or higher are classified as high-risk patients, and this clinical trial sought to confirm the efficacy of the treatment in these high-risk group COVID-19 patients. And the number of days it took to maintain NEWS2=0 for 24 hours was recorded as TTCI=0. As an additional clinical variable, the number of days it took to achieve NEWS2=0 was also recorded.

**[Table 6]**

| physiological parameter | Score | | | | | | |
|---|---|---|---|---|---|---|---|
| | 3 | 2 | 1 | 0 | 1 | 2 | 3 |
| respiration rate (per minute) | ≤8 | | 9-11 | 12-20 | | 21-24 | ≥25 |
| SpO₂ Scale 1(%) | ≤91 | 92-93 | 94-95 | ≥96 | | | |
| SpO₂ Scale 2(%) | ≤83 | 84-85 | 86-87 | 88-92 ≥93 on air | 93-94 on oxygen | 95-96 on oxygen | ≥97 on oxygen |
| Air or oxygen? | | oxygen | | Air | | | |
| Systolic blood pressure (mmHg) | ≤90 | 91-100 | 101-110 | 111-219 | | | ≥220 |
| Pulse (per minute) | ≤40 | | 41-50 | 51-90 | 91-110 | 111-130 | ≥131 |
| Consciousness | | | | Alert | | | CVPU |
| Temperature (°C) | ≤35.0 | | 35.1-36.0 | 36.1-38.0 | 38.1-39.0 | ≥39.1 | |

### <1-3> Examination of clinical therapeutic efficacy of TDCA and antiviral agents for patient with COVID-19 pneumonia

Through clinical evaluation monitoring, efficacy was evaluated by comparing and analyzing the time taken to maintain NEWS2=0 for 24 hours as a clinical indicator, TTCI=0. In particular, given the absence of a definitive treatment for patients with moderate to severe symptoms, including exacerbation of systemic inflammation among COVID-19-infected individuals, additional analysis was conducted on patients with moderate to severe conditions, as indicated by a NEWS2 score of 5 or above, serving as a vital sign of severity.

Antiviral agents primarily used were lopinavir/ritonavir or favipiravir, and the clinical indicators of patients who were administered these two types of antiviral agents in combination were analyzed. Because the number of patients who used remdesivir or umifenovir was very small (less than 5), it was difficult to confirm the effect, so it was excluded from the evaluation.

The results showed, as indicated in Table 1, that the treatment efficacy based on the Recovery Rate Ratio (RRR) by TTCI=0, reflecting comorbidities of moderate severity or higher, was compared among compliant patients (PP) who had received antiviral drug treatment as a baseline factor. The treatment efficacy (RRR) assessed through Cox-regression analysis, considering the baseline factor of antiviral drug administration, was 3.7 with a 'p-value of 0.01', indicating improvement in recovery and statistical significance. Additionally, the treatment efficacy (RRR) examined through Cox-regression analysis for patients with NEWS2 scores of 5 or higher, representing moderate or severe conditions, was 2.7 with a `p-value of 0.02', demonstrating recovery improvement and statistical significance. Based on these findings, when adjusting for antiviral drug administration and the severity of illness as baseline variables through Cox-regression analysis in the entire patient cohort, the treatment efficacy (RRR) was 3.4 with a `p-value of 0.0026', confirming recovery improvement and statistical significance.

In addition, as shown in Figure 2, among the entire clinical patient population (ITT), the clinical remission time based on TTCI=0 was compared using Kaplan-Meier survival analysis for patients who received combination therapy with NuSepin and antiviral drugs such as Lopinavir/Ritonavir or Favipiravir, the results revealed that patients receiving combination antiviral therapy recovered on average at 9.4 days, whereas the placebo group showed a delayed recovery trend, with recovery occurring 3.4 days later at 12.8 days, and this difference was statistically significant with a `p-value of 0.013' confirmed by log-rank test. Additionally, the hazard ratio (HR) assessed through Cox-regression analysis was 3.5 with a `p-value of 0.02', indicating recovery improvement and statistical significance (Figure 2A). When separately analyzing patients with NEWS2 scores of 5 or higher representing moderate or severe conditions, the group receiving combination antiviral therapy recovered on average at 9.8 days, whereas the placebo group showed a delayed recovery trend, with recovery occurring 4.8 days later at 14.6 days, and this difference was statistically significant with a 'p-value of 0.007' confirmed by log-rank test. Moreover, the hazard ratio (HR) assessed through Cox-regression analysis was 4.2 with a 'p-value of 0.01', confirming recovery improvement and statistical significance (Figure 2B).

In addition, as shown in Figure 3, among the entire clinical patient group (ITT), the clinical remission time based on TTCI=0 was compared using Kaplan-Meier survival analysis for patients who received combination therapy with NuSepin and the antiviral drug Lopinavir/Ritonavir, and the results revealed that the group receiving combination therapy with NuSepin and Lopinavir/Ritonavir recovered on average at 8.6 days, whereas the placebo group showed a delayed recovery trend, with recovery occurring 1.1 days later at 9.7 days. When adjusting for the baseline variable of the high-risk age group of 60 years and older through Cox-regression analysis, the hazard ratio (HR) was 7.7 with a `p-value of 0.04', confirming the recovery improvement effect of combination therapy (Figure 3A). When analyzing the time taken for NEWS2 to drop to 0 as an additional evaluation indicator for patients with moderate to severe conditions (NEWS2 score of 5 or higher), the group receiving combination antiviral therapy recovered on average at 9 days, whereas the placebo group showed a delayed recovery trend, with recovery occurring 9 days later at 18 days. This difference was statistically significant with a `p-value of 0.03' confirmed by log-rank test, and the hazard ratio (HR) was 7.7, indicating the recovery improvement effect of combination therapy (Figure 3B).

In addition, as shown in Figure 4, among the entire clinical patient group (ITT), the clinical remission time based on TTCI=0 was compared using Kaplan-Meier survival analysis for the patient group administered in combination with NuSepin and favipiravir as an antiviral agent, and the results revealed that the patient group treated with NuSepin and favipiravir in combination recovered on average at 10.3 days, whereas the placebo group showed recovery at 16 days, 5.7 days later, and the hazard ratio (HR) was 3.8, indicating the recovery improvement effect of combination therapy (Figure 4).

In addition, as shown in Figure 5, among the entire clinical patient group (ITT), the group of patients receiving combination therapy with NuSepin and antiviral drugs (Lopinavir/Ritonavir or Favipiravir) demonstrated a higher proportion of patients returning to normal CRP levels of 10 or below by the 4th day after treatment, at 64.3%, compared to 11.1% in the placebo group, as confirmed by the Wilcoxon Rank sum test, indicating a statistically significant reduction in CRP concentration on the 4th day compared to baseline (Figure 5).

The above results show that the combined administration of TDCA and antiviral agent has an effective treatment effect on patients with COVID-19 pneumonia.

### Industrial Applicability

In the present invention, it was confirmed that clinical symptoms were treated in patients with COVID-19 pneumonia who were administered a combination of a pharmaceutically acceptable salt of taurodeoxycholic acid as an inflammasome inhibitor and an antiviral agent, and thus inflammatory complex inhibitors including taurodeoxycholic acid or a pharmaceutically acceptable salt thereof and antiviral agents, can be used as active ingredients in compositions for the prevention or treatment of lower respiratory tract infectious diseases, including COVID-19.

## Claims

1. A pharmaceutical composition for prevention or treatment of lower respiratory tract infectious diseases, comprising as active ingredients:
a first component containing an inflammasome inhibitor; and
a second component containing one or more antiviral agents, used in complexed, mixed or combination.

2. The pharmaceutical composition of claim 1, wherein the inflammasome inhibitor is taurodeoxycholic acid or a pharmaceutically acceptable salt thereof.

3. The pharmaceutical composition of claim 2, wherein the taurodeoxycholic acid is a compound represented by the Formula 2:

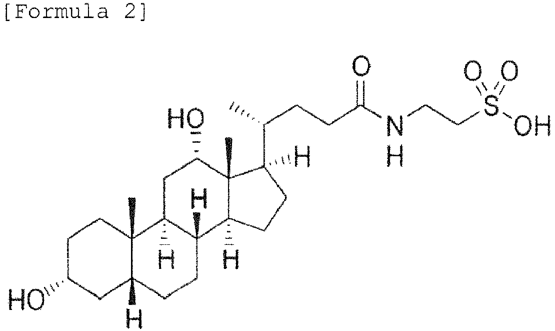

4. The pharmaceutical composition of claim 2, wherein the salt is a sodium salt.

5. The pharmaceutical composition of claim 1, wherein the inflammasome inhibitor is administered at a dosage ranging from 0.01 to 1 mg/kg/day (mg/body weight kg/day).

6. The pharmaceutical composition of claim 1, wherein the antiviral agent is at least one selected from the group consisting of RNA-dependent RNA polymerase inhibitors (RdRp inhibitors), Protease Inhibitors (PIs), Non-Nucleoside Reverse Transcriptase Inhibitors (NNRTIs), Nucleoside Reverse-Transcriptase Inhibitors (NRTIs), Integrase Strand Transfer Inhibitor (INSTI), fusion inhibitor, CCR 5 (Chemokine (C-C motif) ligand 5) inhibitors, flu therapeutic agents, herpes therapeutic agents, hepatitis B therapeutic agents, and hepatitis C therapeutic agents.

7. The pharmaceutical composition of claim 6, wherein the antiviral agent is at least one selected from the group of remdesivir, favipiravir, chlorhexidine, novobiocin, ceftibuten, ribavirin, atovaquone, valganciclovir, cromolyn, bromocriptine, silybin, cefuroxime, fludarabine, galidesivir, sofosbuvir, fenoterol, nitazoxanide, itraconazole, benzylpenicilloyl G, pancuronium bromide, penciclovir, 7-Deaza-2'-C-methyladenosine, idarubicin, diphenoxylate, ganciclovir, tenofovir, tibolone, chenodeoxycholic acid, cortisone, ritonavir, indinavir, nelfinavir, amprenavir, lopinavir, atazanavir, fosamprenavir, tipranavir, darunavir, cobicistat, efavirenz, etravirine, nevirapine, doravirine, rilpivirine, delavirdine, zidovudine, didanosine, zalcitabine , stavudine, lamivudine, abacavir, tenofovir disoproxil fumarate, emtricitabine, tenofovir alafenamide fumarate, saquinavir, dolutegravir, raltegravir, enfuvirtide, maraviroc, elvitegravir, amantadine, rimantadine, oseltamivir, zanamivir, peramivir, baloxavir, laninamivir, aciclovir, valaciclovir, idoxuridine, vidarabine, penciclovir, famciclovir, trifluridine, cidofovir, foscarnet, clevudine, telbivudine, entecavir, adefovir, tenofovir disoproxil, tenofovir alafenamide, besifovir, peginterferon-α-2a, peginterferon-α-2b, boceprevir, dasabuvir, daclatasvir, asunaprevir, elbasvir, grazoprevir, glecaprevir, pibrentasvir, ombitasvir and paritaprevir.

8. The pharmaceutical composition of claim 1, wherein the ratio of the first ingredient containing the inflammasome inhibitor to the second component containing one or more antiviral agents is from 0.00001 to 100 parts by weight of the first component to 1 part by weight of the second component.

9. The pharmaceutical composition of claim 1, wherein the lower respiratory tract infectious disease has at least one symptom selected from the group consisting of fever, cough, phlegm, body aches, sore throat, diarrhea, conjunctivitis, headache, skin rash, difficulty breathing, acute bronchitis, pneumonia, pulmonary purulence, renal failure, renal dysfunction, septic shock, sepsis, and cytokine release syndrome caused by coronavirus infection.

10. The pharmaceutical composition of claim 9, wherein the coronavirus is at least one selected from the group consisting of Middle East respiratory syndrome coronavirus (MERS-CoV), severe acute respiratory syndrome coronavirus (SARS-CoV), and novel coronavirus (SARS-CoV-2) .

11. The pharmaceutical composition of claim 1, wherein the pharmaceutical composition is formulated into a dosage form selected from the group consisting of tablets, capsules, injections, troches, powders, granules, solutions, suspensions, oral solutions, emulsions, syrups, suppositories, vaginal tablets, and pills.

12. A Kit comprising a complexed, mixed, or combination preparation for the prevention or treatment of lower respiratory tract infectious diseases, comprising an agent containing an inflammasome inhibitor; and an agent comprising one or more antiviral agents.

13. The method of claim 12, wherein the complexed, mixed, or combination preparation is formulated into the dosage form selected from the group consisting of tablets, capsules, injections, troches, powders, granules, solutions, suspensions, oral solutions, emulsions, syrups, suppositories, vaginal tablets and pills.

14. A method of the prevention or treatment of lower respiratory tract infectious diseases, comprising the step of administering a first component containing an inflammasome inhibitor; and a second component containing one or more antiviral agents to a subject in complexed, mixed or combination.

15. A use of a first component containing an inflammasome inhibitor; and a second component containing one or more antiviral agents used for a pharmaceutical composition for the prevention or treatment of lower respiratory tract infectious diseases, in a complexed, mixed, or combination preparation for administration to a subject.
